# EUROPEAN PATENT SPECIFICATION

(11) **EP 0 699 304 B1**
(45) Date of publication and mention of the grant of the patent: **01.12.1999**
(21) Application number: 94916795.1
(22) Date of filing: 18.05.1994
(51) Int. Cl.: G01N 31/22, G01N 33/569, B65D 77/24

(54) **DETECTION OF CONTAMINANTS IN FOOD**
NACHWEIS VON VERUNREINIGUNGEN IN NAHRUNGSMITTELN
DETECTION DE CONTAMINANTS DANS LES ALIMENTS

(30) Priority: 19.05.1993 US 64521; 16.02.1994 US 197297
(43) Date of publication of application: 06.03.1996
(73) Proprietor: California South Pacific Investors, Pasadena, CA 91107 (US)
(72) Inventor: GOLDSMITH, Robert, M. Harton Hall, Pasadena, CA 91109 (US); GOLDSMITH, Catherine, H. Harton Hall, Pasadena, CA 91109 (US); WOODAMAN, James, G., Pasadena, CA 91107 (US)
(74) Representative: Williams, John Francis
(86) International application number: US9405511
(87) International publication number: WO9427144

(56) References cited:
- EP-A- 0 069 037
- WO-A-91/19003
- GB-A- 2 234 587
- US-A- 4 285 697
- US-A- 4 746 616

## Description

### Related Applications

This application is a continuation in part of U.S. Application Serial No. 08/064,521 filed May 19, 1993, which is incorporated herein by reference.

### Field of the Invention

The present invention relates to detection of the presence of toxic contaminants in food.

### Background of the Invention

Over the past several years there has been increasing concern over the safety of our food supply. Contamination of food can come from a variety of sources and the type of contamination possible is often dependent on the food involved.

Most animal derived food products, such as raw meat, are exposed to the possibility of contamination before, during or after processing. Such contamination comes from, for example, contact with faecal matter at the slaughter house, from handlers of the food products at any stage of the processing of the food products or from toxins, both naturally occurring and man-made, present in the environment where the food was grown or processed. In most cases, contamination is minor and, if the food is prepared properly, is not a serious threat to the consumer. However, while the contamination of food is generally low, *i.e.*, few bacteria per gram of the food, if the food is not stored under satisfactory conditions or stored for long periods of time, contaminants, such as bacteria, grow to become a serious threat to the eventual consumer of the products. Even if the food products reach the market in an acceptable condition, subsequent treatment by the consumer may lead to the development of serious contamination of the food.

A number of incidents and factors have lead to the growing concern over the food supply. These include:
raw chicken and egg products have been found to be contaminated with *Salmonella* and inadequate cooking of such products has led to serious illness or death of persons who have consumed the contaminated products;
inadequately pasteurized milk products have been found to be contaminated with *Listeria* which has lead to serious illness or death of consumers of the products;
a highly toxic strain of *E. coli* has lead to the death of several people who consumed prepared beef products which had been inadequately cooked;
a number of toxins are known, such as ciguatoxins, which contaminate fish. These toxins are not inactivated or destroyed by cooking and so their presence in fish is a threat to any consumer of the product;
shell fish, such as oysters, concentrate any contaminants present in the water in which they grow and, since they are frequently eaten raw, pose a threat to the health of consumers; and
fish is increasingly eaten raw which adds to the possibility of increased outbreak of illness from water borne contaminants.

The only means the consumer has of determining if the food they purchase is contaminated is by visual inspection and by smell. These are usually inadequate to detect contamination.

US-A-4,285,697 discloses a food spoilage indicator comprising a liquid crystal disposed in a carrier of plastic tape, at least one portion of which is semi-permeable to gases generated in food spoilage. US-A-4,746,616 describes a method for treating consumable products, such as foods, drugs or dietary supplements, for example drugs enclosed in capsules, to detect contamination. A colorimetric indicator, which can react with a contaminating substance such as cyanide, is incorporated into the consumable product or the packaging for the product. When the indicator comes in contact with the contaminating substance, a readily detectable colour signal is produced.

There is a need for a reliable way to detect if a food product purchased by a consumer is fit for consumption. Any solution to this problem should be relatively inexpensive and able to detect a number of agents capable of causing illness. It should also be simple to "read" so that a consumer, who does not have access to sophisticated testing equipment or specialised knowledge, can readily determine if the products they have purchased are free from contamination.

According to the present invention, there is provided a food contamination detector as specified in claim 1, a food product package as specified in claim 22, and methods as specified in claims 27 and 31.

The present invention relates to a food contamination detector. The food contamination detector comprises an indicator bound to a substrate. The indicator is in communication with juices from food which are to be tested for the presence of a toxin.

A means for changing the colour of the indicator when the toxin is present in the juices from the food is provided to indicate that the food is contaminated. In one embodiment of the invention the means for changing the colour comprises a labeled antibody which dissociates from the substrate in the presence of a toxin. In another embodiment the means for changing colour comprises a labeled antibody which binds to the substrate in the presence of a toxin. In another embodiment the change in colour results in a change in a bar code.

### Brief Description of the Drawings

These and other features and advantages of the present invention will be better understood by reference to the following detailed description when considered in conjunction with the accompanying drawings in which:
FIG. 1 is a top view of a packaged food product;
FIG. 2 is a bottom view of the packaged food product with a bar code detector system;
FIG. 3 is a side sectional view of the packaged food product showing the bar code detector system in the package;
FIG. 4 is one embodiment of the bar code detector system of the present invention, prior to attachment to a food package;
FIG. 5 is a schematic diagram of a bar code reader for use in the present invention;
FIG. 6 is a side sectional view of another embodiment of the bar code detector system in a package tray without food;
FIG. 6A is an enlarged view of part of FIG. 6;
FIG. 7 is a perspective view of the bottom of the absorbent liner of FIG. 6 with one component of the bar code system attached;
FIG. 8 is a front view of the component shown in FIG. 7;
FIG. 9 is a front view of another component of the bar code system of FIG. 6;
FIG. 10A is a front view of the components of FIGs. 8 and 9 as they appear from the outside of the food package in the absence of contamination;
FIG. 10B is a front view of the components of FIGs. 8 and 9 as they appear from the outside of the food package in the presence of contamination;
FIG. 11 is a side sectional view of another embodiment of a bar code detector system in a package tray without food;
FIG. 12 is a perspective view of a liner for use in a variation of the bar code system of FIG. 11;
FIG. 13 is a perspective view of a carcass indicator strip incorporating a principle of the invention prior to reaction with toxins; and
FIG. 14 is a perspective view of the carcass indicator strip of FIG. 13 after reaction with toxins.

### Detailed Description of the Specific Embodiments

The present invention uses an indicator which may be in the form of words, symbols or part of a bar code that identifies the product at point of purchase, sale, or distribution as a detector system for toxins and other contaminants that may be present in food products. As used herein toxin means chemicals or pathogenic organisms which may be transferred from food to the consumers of the food, or other agents which may be toxic or result in illness in the consumer of the contaminated food products.

The invention is described in the context of bar codes because this is currently the prevalent way to identify food products, including information about product type, quantity, price, unit price, and origin in a machine readable manner. The invention is applicable, however, to other product identifying systems, machine readable and/or readable to a human. When the term "visible" is used herein, it means visible or readable by a bar code reader or other scanning apparatus.

The same reference numbers are used throughout the drawings to identify similar parts or elements.

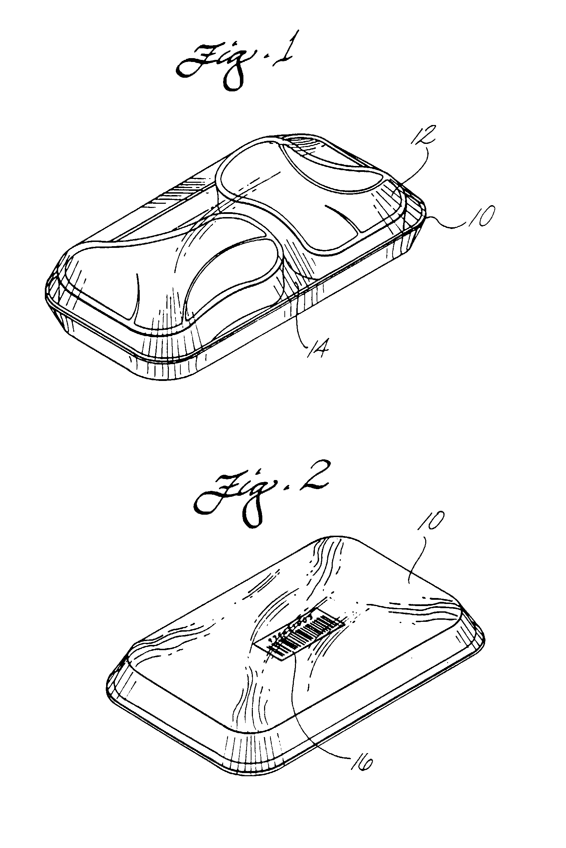

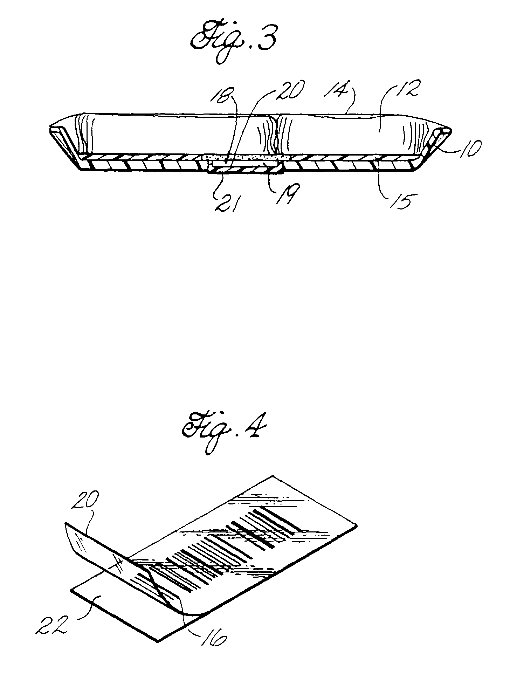

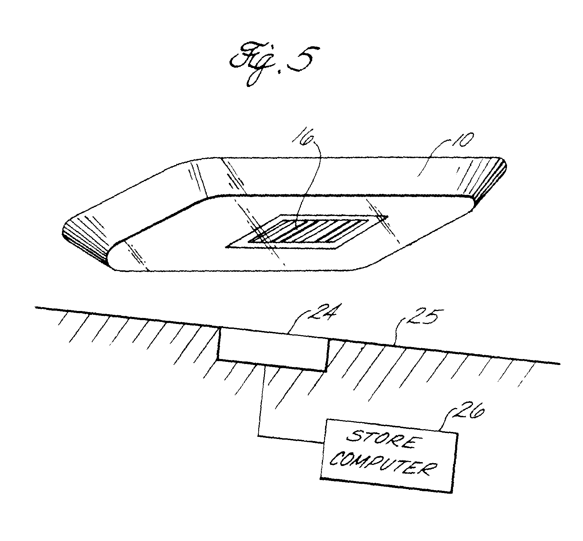

Food products are often "mass produced" and sold at retail outlets in prepackaged containers such as that illustrated in FIGs. 1-3. Typically, such packages include a styrofoam tray 10 which contains the food product 12. The tray and food are sealed in a transparent plastic wrap material 14. An absorbent pad 15 lies between food product 12 and the inside bottom of tray 10.

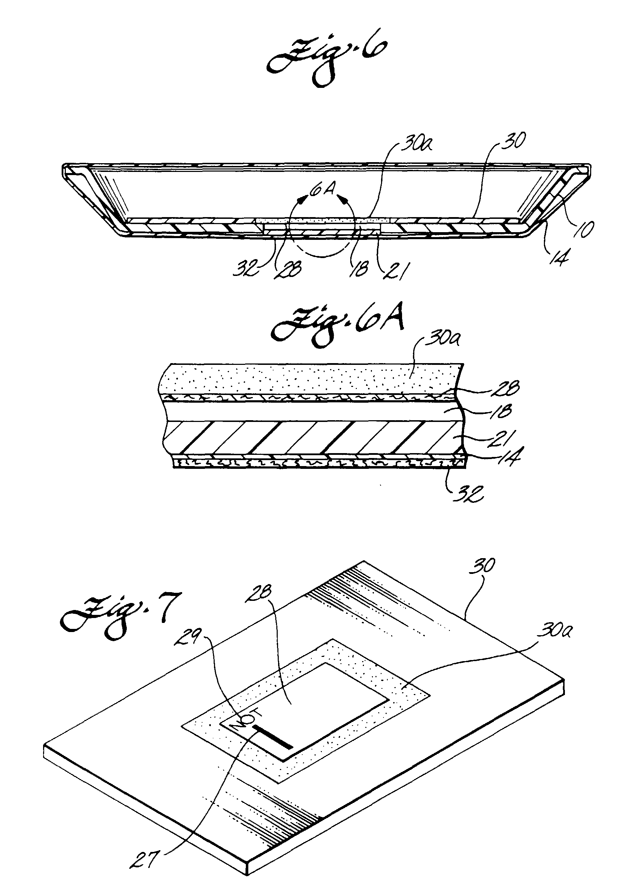

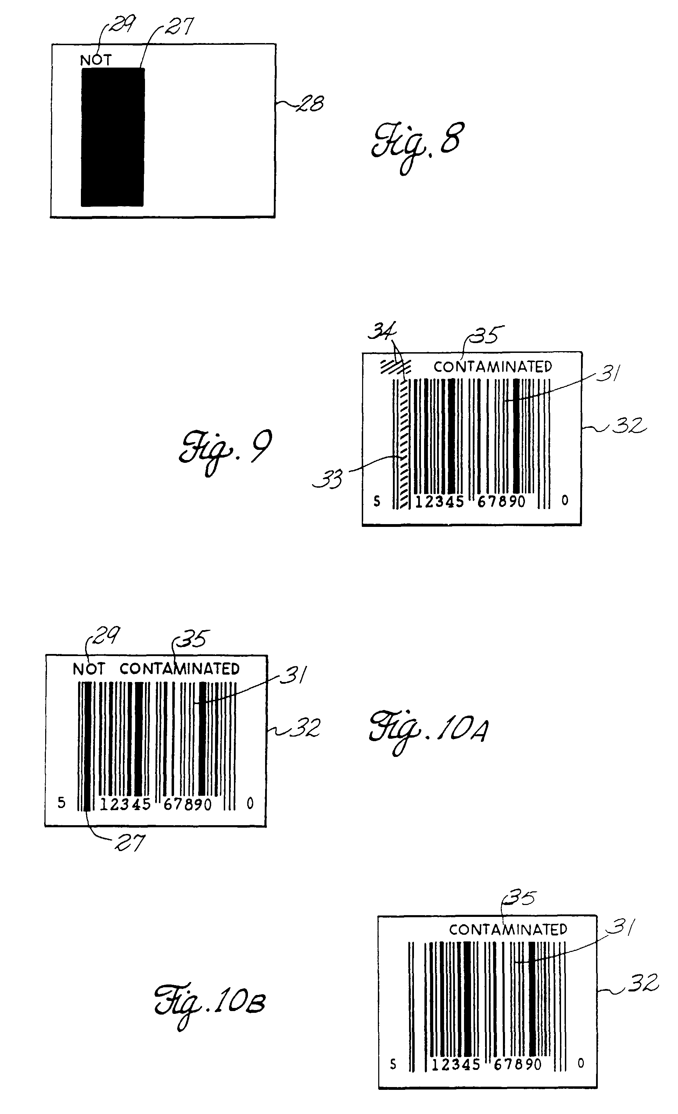

A bar code system 16 is used on the products for scanning at the check-out register (FIG. 5), to reduce errors in totaling purchases and for stock control. The bar code system comprises a series of bars which represent a number, identifying the product. In the practice of the present invention the product identifying system, e.g., the bar code system, also serves the purpose of detecting toxins in the food products.

In the embodiment of FIGs. 1-3, bar code system 16 is printed on a transparent membrane or substrate 20. One side of substrate 20 has a self-adhesive surface for attachment to the interior of tray 10 and the other side of substrate 20 has printed on it bar code system 16. The bottom of styrofoam tray 10 has a rectangular hole 18. Hole 18 is covered by a window 21 formed by a transparent sheet of material such as MYLAR® (a trademark of DuPont) using a suitable adhesive to seal the MYLAR to the styrofoam material. Hole 18 and window 21 also serve as a collector 19 for liquids and juices from food product 12 so the latter can come into contact with bar code system 16. Substrate 20 can be prepared with a peelable, protective release layer 22 (FIG. 4), which covers bar code system 16 prior to its application to a package. At the site of packaging of food product 12, release layer 22 is peeled off and the adhesive side of substrate 20 is placed on the inside surface of window 21 so that bar code system 16 faces the interior of the package and is exposed to the juices of food product 12. Alternatively, substrate 20 could also serve as the window, in which case it would be attached to, cover, and seal hole 18.

The bar code system is formed by labeled antibodies bound to antigens. The labeled antibodies function as an "ink" and are "printed" in a bar code pattern on the transparent substrate 20. First, the antigens are bound to the entire surface of substrate 20 or the portion of its surface on which the bar code system is to be placed. Then, the bar code system is applied to the antigen coated surface of substrate 20 by a bar code printer, using the labeled antibody as the ink. Preferably, bar code system 16 serves the normal product identifying function of a bar code, i.e., it represents price, price per unit, type of product, origin, and quantity or weight information. As illustrated in FIG. 5, food packages carrying bar code system 16 are passed under a bar code scanner or reader 24 mounted on a counter 25 at the point of sale to read the product information in the usual way. A store computer 26 processes this information to totalize the amount of purchase and to manage inventory.

The bar code system for use in the invention is prepared by irreversibly binding an antigenic determinant of toxins or contaminants of interest to the transparent substrate. The antigenic determinant may be a small portion of the toxin, which is specific for that toxin, it may be the toxin itself, an analog of the toxin or other compound which is capable of "mimicking" the toxin, or pathogenic microorganisms, all of which are referred to herein as "toxins." Substrates suitable for binding the toxin are well known in the art. If substrate 20 serves as window 21 it must be impervious to the food juices. Suitable substrates include substrates such as those made from activated hydrophobic polyvinylidene, polyvinylidene difluoride, mixed esters of cellulose nitrate and cellulose acetate, hydrophobic polyvinylidene difluoride, hydrophilic polyvinylidene difluoride, laminated and unlaminated polytetrafluroethylene, microfiber glass, cellulose and polypropylene. Once toxins are bound to the substrate other binding sites, which remain on the substrate, are blocked by contacting them with an "inert" binding agent such as bovine serum albumin or other suitable blocking agent.

Once the toxin is bound to the substrate a labeled antibody, which exhibits a specificity for the toxin, also referred to herein as anti-toxin, is bound to the toxin. Antibodies suitable for use in the present invention include monoclonal and polyclonal antibodies. The preparation of such antibodies, specific for a desired toxin, are well known in the art. In some cases it may be necessary to conjugate the toxin to a protein to "mask" the toxicity of the antigen. Otherwise injection of the toxic antigen may result in the death of the animal in which the antibodies are to be prepared. Methods of conjugating compounds are well known in the art and one such method is described by Hokama *et al., Mycotoxins and Phycotoxins '88,* A Collection of Invited Papers at the Seventh International IUPAC Symposium of Mycotoxins and Phycotoxins, Tokyo, Japan 1988, pp. 303-310 (Elsevier Science Publishers, Amsterdam), which is incorporated herein by reference.

In one embodiment of the present invention the antibody is labeled with a colored latex bead. The preparation of antibodies labeled with colored latex beads is well known in the art. Such labeled antibodies may be prepared by diluting latex beads in a solution such as phosphate-buffered saline (8.1 mM Na₂HPO₄, 1.5 mM KH₂PO₄, 137 mM NaCl, 1.6 mM KCl) and mixing the solution gently to suspend and distribute the latex beads in the solution. Preferably, about a 10% (wt/v) suspension of latex beads is diluted about 1:100, to give a suspension of about 0.1% (wt/v) latex beads. An antibody solution is added to the latex bead suspension. Preferably, about 0.3 to about 0.6 mg of antibodies are added for each mg of latex beads, however, this ratio will vary depending on the specificity and sensitivity of the antibody preparation and the type of support being used. The amount of antibody to be used for the preparation of labeled antibodies is derived experimentally, using different dilutions of the antibody preparation. After addition of the antibody, the solution is gently mixed and incubated at about 4°C for about 16 to about 20 hours. At the completion of the incubation, the labeled antibodies are washed with phosphate-buffered saline, and the sensitivity and specificity of the labeled antibody preparation are tested.

The sensitivity and specificity of the labeled antibodies are tested by coating a substrate with a preselected amount of toxin. When contacted with the labeled antibody, the labeled antibody binds to the toxin, resulting in the development of the desired color on the substrate. The color which develops will not be washed off by rinsing in a solution such as phosphate-buffered saline. Binding of the antibody to the toxin results in the development of color for the bar code pattern forming a bar code detector system named by the owner of this invention the SIRA BAR™ system. In effect, the labeled antibodies act as a type of "ink" so the bar code pattern can be visualized.

In use with raw meat products, the bar code detector system is exposed to juices from the meat. The juices collect in the collector and come in contact with the bar code system. If a toxin is present in the juices, the antibodies will release from the bar code pattern and bind to the toxins present in the juices, thus altering or destroying the bar code pattern. Such antibody type assays are in and of themselves well known in the art and are referred to as competitive assays.

A consumer can detect the presence of the toxin in the food product by a visual inspection of the bar code system. If the consumer does not notice the alteration of the bar code, it is detected by bar code reader 24 at the check-out counter (FIG. 5) because store computer is configured to emit an alarm to warn that a altered bar code system has been detected. The contaminated products can then be replaced with non-contaminated products.

A labeled antibody is one means of indicating the presence of a toxin or other contaminant in the juices of a food product. Those skilled in the art will be aware of other indicators such as chemical indicators, which are useful in the practice of the present invention. Instead of destroying the bar code, the bar code could be altered in some other way, e.g., by change of color, depending on the nature of the indicating system. In general, the alteration of the bar code is detectable by the bar code reader so contamination of products can be automatically determined by the electronics. Thus, the invention presents a format or vehicle to utilize existing toxin or contaminant indicating systems more effectively.

The bar code reader can also be used to indicate whether packaged products are in satisfactory condition at the time they left the supplier. If contaminated products are detected in the processing stream, the supplier can find out the source of contamination and implement remedial steps to ensure that the source of contamination is eliminated.

The same toxin could be used for all the bars of the bar code system or one or more toxins could be used for different bars. In this way a number of contaminants or toxins, that are commonly associated with a particular food, can be detected by a single bar code system. The bar code system would not only indicate that the food was contaminated but would also indicate the type of contamination.

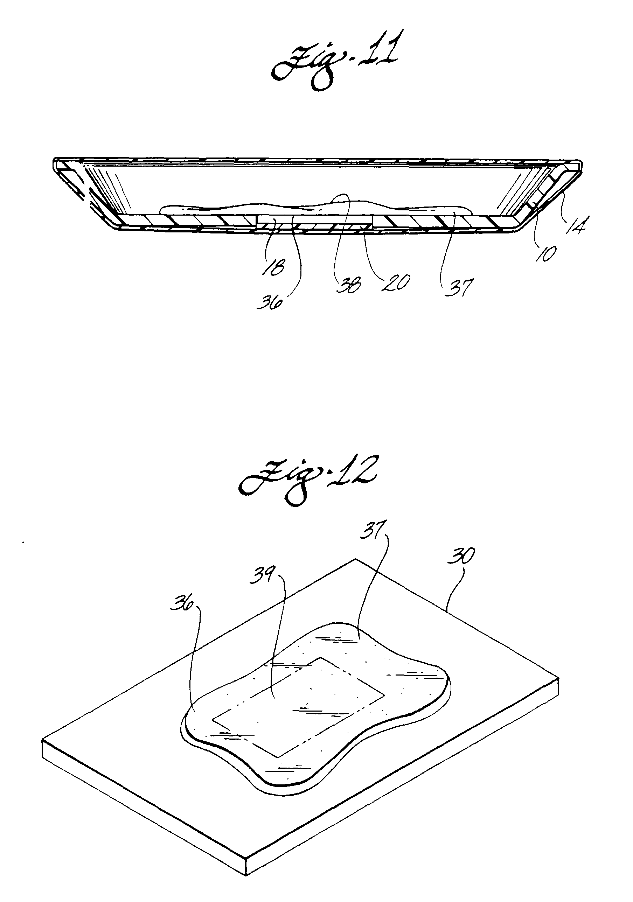

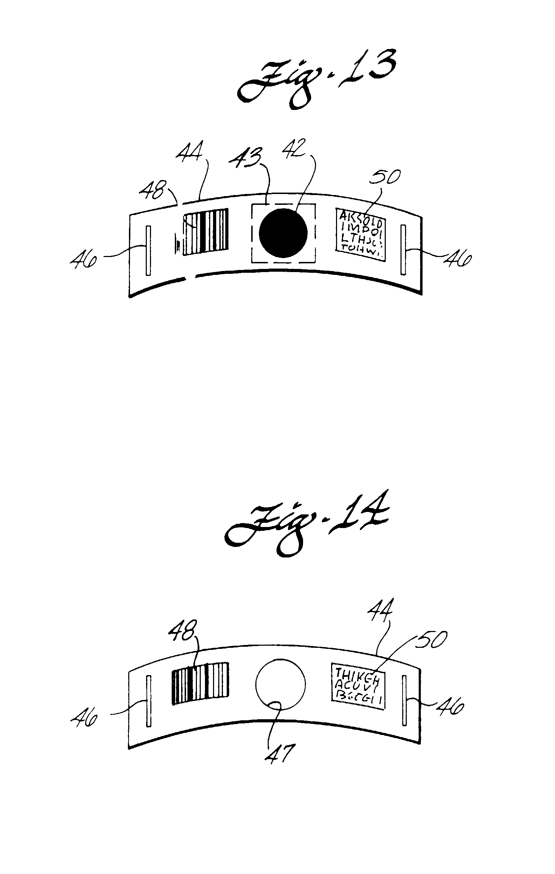

In another embodiment of the present invention shown in FIGs. 6-10, the contamination indicator is incorporated in a bar code system having two components--one component inside the package and another component outside the package. A substrate 28 is attached to the bottom of an absorbent liner 30. Such liners are well known in the art. The liner is an absorbent material that draws juices and other fluids away from the meat to the surface of substrate 28. Substrate 28 is preferably pervious to the juices of the food product, but it does not need to be transparent. The position of substrate 28 on liner 30 is precisely set. As illustrated in FIGs. 7 and 8, one component of the bar code system comprises visible indicator elements 27 and 29 printed on the exposed surface of substrate 28. Indicator elements 27 and 29 may include a bar, a symbol, letters, or a combination thereof. In the illustrated embodiment indicator element 27 comprises a bar, given the trademark SIRA BAR™ by the owner of this invention, and indicator element 29 comprises the word "NOT". Indicator elements 27 and 29 are printed on substrate 28 using labeled antibodies as "ink", as described above.

In this embodiment, the bottom of styrofoam tray 10 has a window 21 formed by a transparent sheet of material such as MYLAR® (a trademark of DuPont) using a suitable adhesive to seal the MYLAR to the styrofoam material. The liner and tray are designed so the liner can be precisely positioned in the bottom of the tray. For example, liner 30 could be dimensioned so that when it is placed in tray 10 it fills the bottom of the tray with substrate 28 in register with window 21. In this way, the close fit between liner 30 and tray 10 serves to insure that indicator elements 27 are precisely positioned with respect to the second component of the bar code system, which is placed on the exterior of the bottom of tray 10 and wrap material 14. Alternatively, ridges (not shown) could be molded into the inside bottom surface of tray 10 to position liner 30 precisely and hold it in place.

As illustrated in FIG. 9, the second component of the bar code system comprises a word 35 and a plurality of bars 31 printed on an opaque substrate 32 with ordinary ink and cut out sections 33 and 34 die cut from substrate 32. Section 33 is smaller than bar indicator element 27. Section 34 is larger than word indicator element 29. Bars 31 perform the normal product identifying function of a bar code, i.e., they represent price, unit price, type of product, origin, and weight or quantity. Substrate 32 has the same dimensions as window 21 and is placed on the outside of wrap material 14 so substrate 32 coincides with window 21. As a result, the position of substrate 32 is precisely set relative to substrate 28 so that indicator elements 27 and 29 are aligned with cut outs 33 and 34, respectively, and are normally visible from outside the package. Indicator element 27 completely fills cut out section 33 and indicator element 29 fits totally within cut out section 34. In the illustrated embodiment, word 35 is "CONTAMINATED".

When substrates 28 and 32 are aligned, the first and second components fit together to form the bar code system. As illustrated in FIG. 10A, the words "NOT CONTAMINATED" are visible from the exterior of the package and indicator element 27 and bars 31 can be read by a bar code reader when no contaminants are present in the food juices inside the package. When contaminants are present, the labeled antibodies from which indicator elements 27 and 29 are formed react with the toxin and are removed from the substrate 28. As illustrated in FIG. 10B, this leaves only word 35 and bars 31 visible. In the absence of element 27, the bar code reader senses that the bar code system is "defective" and in the absence of element 29 humans can visually observe that the contents of the package is "CONTAMINATED".

Since it is desirable to detect different toxins in different food products it is also desirable, to place indicator element 27 in different locations on substrate 28 and cut out 33 in different locations on substrate 32 aligned with the locations on substrate 28, depending upon the toxin to be detected.

The described two component bar code system can be used to great advantage with the conventional bar code applicator machines used to mark food products in supermarkets. Such machines have a conveyor on which wrapped food packages are transported past a weighing station and a bar code label application station into a temporary storage bin. At the label application station a label carrier roll is fed past a printer where the product information is printed on the bar code labels (substrate 32) and under a blade where the bar code labels are released from the carrier and picked up by one or more robot arms for delivery to the packages. A worker punches a product identification code into a key pad. A controller calculates from the product identification code and from the weight the product information to be printed on the label such as price, weight, unit price, and historical data, i.e., origin, and controls the printer to print the bar code pattern and alphanumeric characters on the labels. The controller coordinates, i.e., times, the operation so the labels are applied to the proper packages.

A preferred method will now be described for using the two component bar code system with a modification of the conventional bar code applicator machines used to mark food products such as meat, poultry, or fish, in supermarkets. In a central processing plant, indicator elements 27 and 29 are printed on substrates 28 with a labeled antibody or other contaminant detector as ink; then substrates 28 are mounted on liners 30 in a precise relative position and packed in shipping cartons. Liners are so prepared in separate cartons for each of a number of different toxins or contaminants and tray sizes. The cartons are shipped to the supermarkets or packaging facility where the food products are packaged in trays, wrapped, and bar code labeled with the bar code applicator machine. The packaging operation takes place in the following order--
1. For each different toxin or contaminant, one of the corresponding liners is placed in a tray sized for the particular liner.
2. The food product is placed in the tray.
3. The food product and tray are covered with the wrap material.
4. The package is placed in a bar code applicator machine and the product identification code is entered through the keyboard.
5. The package is weighed in the machine and transported by the conveyor to the label application station.
6. The bar code applicator machine is modified to incorporate a label cutting die or die set in the path of the carrier between the roll and the printer. The die is adjustable in position and its position is set by the controller depending upon the particular product identification code. Each time a bar code label passes the die, the die is actuated by the controller to form the die cut sections (33 and 34 in FIG. 9).
7. The printer is operated by the controller to print words 35 and bars 31 on the bar code labels with ordinary ink.
8. The bar code labels are applied by the machine to a precise location on the outside of the packages in alignment with substrates 28 (FIG. 6).

In summary, the first component of the bar code system, which requires tight manufacturing controls, is pr duced at a central processing plant. At the supermarket, workers without any special skill can reliably incorporate the first component into food product passages and add the second component of the bar code system in the usual way, i.e., with a bar code applicator machine. The only special training for the workers at the supermarket is the proper selection and placement of the liner (30 in FIG. 6). If a worker makes a mistake in selection or placement of a liner, bar 27 is not aligned with cutout 34 and the bar code reader senses the mistake. This provides a check to ensure that the correct toxin detecting bar has been used with the correct food product.

Substrate 32 is preferably opaque and white, or at least light in color to create a strong contrast with the bar codes, which are preferably printed in a dark color. For this reason cutouts 33 and 34 are required so substrate 32 does not hide visual elements 27 and 29 of substrate 28. If sufficient contrast is otherwise available, substrate 32 can be transparent and the cutouts can be eliminated.

In the embodiment of FIG. 11, the contamination indicator is also incorporated in a bar code system having two components--one component inside the package and another component outside the package. One component comprises a transparent bag 37 constructed from a bottom panel 36 and a top panel 38. Bag 37 is placed over hole 18 and the bottom panel 36 is secured to tray 10 by adhesive to seal hole 18 and form a window. Bottom panel 36 is fabricated from a substrate that is impervious to the food juices. A first antibody against the toxin of interest is bound to an area of the interior surface of bottom panel 36 identical in size and shape to or larger than hole 18. Top panel 38 is fabricated from a semipermeable membrane. The top and bottom panels are sealed together at their edges by use of an adhesive or other suitable method such as heat, to form a sealed bag, i.e., bag 37. Prior to sealing the bag a solution including a labeled second antibody against the toxin of interest is introduced into the bag. Although the first and second antibodies could be the same, they are preferably different. Thus, the second antibody preferably recognizes different antigenic determinants on the toxin than the first antibody. The second antibody is labeled with an indicator such as a colored latex bead so that the resultant labeled antibody is of a large size. The labeled antibody, present in the solution, is at a dilute concentration so that light will readily pass through the solution and so that little or no color is discernable.

The semipermeable membrane has a pore size which is large enough to allow the toxin of interest to enter the bag, but which is small enough to prevent the labeled antibody from leaving the bag. Such membranes are well known in the art and are commercially available in a variety of pore sizes. The pore size of the semipermeable panel is selected so that the toxin of interest will pass through the semipermeable panel to the interior of the bag.

When a toxin is present in the juices of a meat product packed in the tray, the toxin passes into the bag through semipermeable panel 38 and binds to antibodies bound to panel 36. The toxin also binds to the labeled second antibody present in the solution in the bag. As a result, panel 36 becomes colored by the sandwich assay of the first antibody, the toxin, and the labeled second antibody, thereby indicating the presence of a toxin in the juices.

The second component comprises bar code system 16 printed on substrate 20, which is a transparent material such as MYLAR®. Substrate 20 is placed over hole 18 on the exterior of the meat tray, and preferably outside wrap material 14. When a toxin is not present in the juices panel 36 remains clear and the bar code system can be easily read against the clear background. When a toxin is present in the juices, the toxin binds to panel 36 and to the labeled antibody such that the substrate background becomes densely colored. In a preferred embodiment the color of the beads used is black and the uncolored background is white or clear. The dense color of the first component prevents the bar code of the second component from being distinguished from the background by the bar code reader. This effectively obliterates or changes the bar code system and indicates that the food contained in the package is contaminated.

A variation of the two component bar code system of FIG. 11 is partially illustrated in FIG. 12. Panel 36 is secured to the underside of liner 30 using an adhesive or other suitable means of attachment. The liner is an absorbent material that draws juices and other fluids away from the meat to the surface of semipermeable panel 38 and serves to align bag 37 with hole 18, in the manner described in connection with FIG. 6. The juices pass through the semipermeable panel and into the interior of bag 37. On the interior of surface of panel 36 antibodies are attached as described above. The antibodies are attached to a rectangular area 39 on the inside surface of panel 36 such that when the liner is placed in the food tray rectangular area 39 aligns with hole 18. Substrate 20 is attached to the outer surface of tray 10 after tray 10 has been covered with wrap material 14. A bar code system is printed on substrate 20 by the bar code applicator machine. The presence of toxins are then detected as described above.

In another embodiment of the invention shown in FIGs. 13 and 14, a symbol such as a colored dot 42 is printed on a porous substrate 40. Substrate 40 is designed to be attached to the surface of a beef carcass or other bulk food product to determine if the carcass is contaminated. The "ink" used to print the dot is labeled antibodies attached to toxin as described above. In the illustrated embodiment, substrate 40 is in two parts--a porous indicator strip 45 and an opaque holder strip 44 that covers and secures the indicator strip in place. The labeled antibody is bound to a square area 43 of the indicator strip. Substrate 40 comprises flexible material which fits the contour of the carcass and keeps the indicator strip in contact with the surface of the carcass. A circular hole 47 is cut in the holder strip and area 43 aligns with the hole, so that when the substrate and holder strip are attached to the carcass colored dot 42 appears. If toxins are present in the meat of the carcass the antibody becomes unbound from area 43 and the dot disappears to indicate the presence of toxins in the carcass. Substrate 40 is attached to the carcass by use of stainless steel staples 46. The holder strip may also be used to display other identifying information, such as a bar code system 48 and printed matter 50. Bar code system 48 and printed matter 50 could be printed with ordinary ink.

The present invention is not to be limited to the specific embodiments shown which are merely illustrative. Various and numerous other embodiments may be devised by one skilled in the art without departing from the spirit and scope of this invention. For example, with respect to the embodiments of the present invention illustrated in FIGs. 6-12, while the invention is described for use with antibodies against a single toxin, mixtures of antibodies, against a number of different toxins could be used. With the use of different antibodies, multiple, different toxins which could be present in the meat sample can be detected. Also, while the invention is described primarily in relation to obliterating a bar code, the antibody bound to the substrate could also be in the form of a symbol or wording which appears, or disappears depending on the type of antibody-toxin "assay" used. Such a symbol or wording could be read without the aid of a bar code reader. Also while some embodiments are described in conjunction with a liner, these bar code systems could also be used in the absence of a liner. Similarly, embodiments described without a liner could be used in conjunction with a liner. The scope of the invention is defined in the following claims.

## Claims

1. A food contamination detector comprising a food container (10); an indicator (16) comprising an antibody attached to a substrate (20) in communication with juices from the food; means (36, 38) for changing the appearance of the indicator when a toxin is present in the juices from the food.

2. A food contamination detector as recited in claim 1, wherein the means (36, 38) for changing the appearance of the indicator is operative to change the colour of the indicator.

3. A food contamination detector as recited in claim 1 or 2, wherein the means (36, 38) for changing the appearance of the indicator comprises a label attached to the antibody such that the antibody dissociates from the substrate in the presence of the toxin.

4. A food contamination detector as recited in claim 3, wherein the label comprises a coloured latex bead.

5. A food contamination detector as recited in any preceding claim, wherein the indicator is a bar code (31) or symbol that becomes illegible when the toxin is present.

6. A food contamination detector as recited in claim 1, wherein the indicator comprises a first antibody attached to a transparent substrate (20); and a solution in contact with the first antibody attached to the transparent substrate; wherein the solution comprises a labelled second antibody which becomes bound to the transparent substrate rendering the substrate opaque in the presence of a toxin in the juices of the food.

7. A food contamination detector as recited in claim 6, wherein the solution comprising the second antibody is enclosed in a compartment.

8. A food contamination detector as recited in claim 7, wherein the compartment comprises a semi-permeable membrane (38).

9. A food contamination detector as recited in claim 6, 7 or 8, wherein the first antibody is able to recognize an antigenic determinant on the toxin which is different from the antigenic determinant on the toxin recognized by the second antibody.

10. A food contamination detector as recited in any one of claims 6 to 9, comprising a bar code (31) aligned with the transparent substrate (20).

11. A food contamination detector as recited in any one of claims 6 to 10, wherein the transparent substrate seals a hole (18) in a food container (10).

12. A food contamination detector specified in claim 1, wherein the indicator (34) includes markings which are removed in the presence of a toxin, the detector comprising a bar code (31) aligned with the indicator such that the combination of the presence of markings on the indicator and the bar code forms an intact visible indicator.

13. A food contamination detector as recited in claim 12, wherein the markings on the indicator are formed by labelled antibodies bound to a substrate.

14. A food contamination detector as recited in claim 12 or 13, wherein removal of the markings from the indicator strip results in a defective indicator.

15. A food contamination detector according to claim 1 comprising :
a toxin bound to the substrate (20); and wherein the labelled antibody is printed thereon to form a bar code such that when the detector is contacted with a toxin specific for the indicator, the labelled antibodies are removed from the substrate, destroying the bar code and thereby indicating the presence of the toxin in the food.

16. A food contamination detector as recited in claim 1 or 15, wherein the toxin is selected from the group consisting of chemicals, pathogenic organisms or other illness inducing toxic agents.

17. A food contamination detector as recited in claim 15 or 16 wherein the substrate (20) comprises a transparent membrane (20).

18. A food contamination detector as recited in claim 15, 16 or 17, wherein the indicator comprises an antibody bound to a coloured latex bead.

19. A food contamination detector as recited in claim 1 or 18, wherein the antibody is selected from the group consisting of monoclonal antibodies and polyclonal antibodies.

20. A food contamination detector as recited in any one of claims 15 to 19, wherein first and second toxins are bound to the substrate at first and second positions respectively; first and second indicators being bound to the first and second toxins respectively, the first and second indicators forming parallel bars on the substrate and where the presence of a food sample containing a contaminant specific for the indicators will cause the indicators to become unbound and efface the parallel bar pattern.

21. A food contamination detector as recited in claim 20, wherein additional toxins are printed on the substrate and bound to the indicators to form a bar code, changes in which are detectable by a bar code scanner.

22. A food product package comprising a food contamination detector, a transparent wrap (14) covering the container and the food product; a first product information component disposed inside the wrap so it is visible from the exterior of the package and comprising a first substrate on which one or more first visual elements (31) are printed with a toxin detecting material according to claim 1; and a second product information component disposed outside the wrap and comprising a second substrate aligned with the first substrate without hiding the first visual elements and a plurality of second visual elements (35) printed on the second substrate with a non-toxin detecting material.

23. A package as in claim 22, which the container (10) has a window (18) formed in a bottom panel thereof, the first component comprises an absorbent liner (38) dimensioned to fit in the container in a predetermined position relative to the window, and the first substrate is attached to the bottom of the liner in alignment with the window when the liner is in a predetermined position.

24. A package as in claim 23, in which the second substrate is aligned with the window and the first substrate.

25. A package as in claim 24, in which the second substrate is opaque and has one or more cutouts, the one or more visual elements of the first substrate being visible through the one or more cutouts.

26. A package as in any one of claims 22 to 25, in which the first and second elements together form a bar code (31).

27. A method of detecting contamination in food comprising the steps of placing food that creates food juices in a container (10); forming an indicator (16) by attaching an antibody to a substrate (20); placing in contact with the juices in the container the indicator (16), the appearance of which changes in the presence of a toxin; and noting the appearance of the indicator to determine if the food is contaminated.

28. A method as recited in claim 27, wherein the step of placing an indicator in contact with the juices comprises stapling the indicator directly to the food to be tested.

29. A method as recited in claim 27 or 28, wherein the forming step attaches a coloured latex bead as a label.

30. A method as recited in any one of claims 27-29, wherein the indicator is a bar code (31) and the change in appearance of the indicator makes the bar code unreadable by a bar code scanner.

31. A method according to claim 27 comprising the steps of binding toxins to the substrate (20); printing labelled antibodies thereon to form a bar code; contacting the bar code with material derived from the food which is suspected of being contaminated with a toxin; and scanning the bar code to determine the presence of contaminants in the food.

32. A method as recited in claim 31, wherein the toxins are selected from the group consisting of chemicals, pathogenic organisms or other illness-inducing toxic agents.

33. A method as recited in claim 31 or 32, wherein the substrate is provided with a transparent membrane.

34. A method as recited in claim 31, 32 or 33, wherein the indicator comprises an antibody bound to a latex bead.

35. A method as recited in claim 34, wherein the antibody is selected from the group consisting of monoclonal antibodies and polyclonal antibodies.

36. A method of packaging a food product comprising the steps of: forming an indicator (16) by printing one or more visible toxin indicating elements (16) on a first substrate, including the substep of attaching a labelled antibody to the substrate (20), to form a material that visibly changes when subjected to a toxin to be detected; placing the first substrate (20) in the food container; placing a food product in the container; wrapping the container and the product in a sheet (14) of transparent material so the first substrate is visible; printing a plurality of visible product identifying elements on a second substrate; and applying the second substrate to the exterior of the sheet in alignment with the first substrate without hiding the one or more toxin indicating elements.

37. A method as in claim 36, in which the step of placing the first substrate in a food container comprises attaching the first substrate to one surface of an absorbent liner (38) and placing the one surface of the liner in contact with the bottom of the container in a predetermined position, and forming a window (18) into the bottom of the container in alignment with the first substrate.

## Patentansprüche

1. Nahrungsmittelverunreinigungs-Detektor, umfassend einen Nahrungsmittelbehälter (10); einen Indikator (16), der einen mit einem Substrat (20) verbundenen Antikörper umfaßt und mit Säften aus dem Nahrungsmittel in Verbindung steht; ein Mittel (36, 38) zum Verändern des Erscheinungsbilds des Indikators, wenn ein Toxin in den Säften aus dem Nahrungsmittel anwesend ist.

2. Nahrungsmittelverunreinigungs-Detektor nach Anspruch 1, wobei das Mittel (36, 38) zum Verändern des Erscheinungsbilds des Indikators so wirkt, daß es die Farbe des Indikators verändert.

3. Nahrungsmittelverunreinigungs-Detektor nach Anspruch 1 oder 2, wobei das Mittel (36, 38) zum Verändern des Erscheinungsbilds des Indikators eine mit dem Antikörper verbundene Markierung umfaßt, so daß sich der Antikörper bei Anwesenheit des Toxins von dem Substrat löst.

4. Nahrungsmittelverunreinigungs-Detektor nach Anspruch 3, wobei die Markierung eine gefärbte Latex-Perle umfaßt.

5. Nahrungsmittelverunreinigungs-Detektor nach einem der voranstehenden Ansprüche, wobei der Indikator ein Strichcode (31) oder ein Symbol ist, das unleserlich wird, wenn das Toxin anwesend ist.

6. Nahrungsmittelverunreinigungs-Detektor nach Anspruch 1, wobei der Indikator einen mit einem transparenten Substrat (20) verbundenen ersten Antikörper umfaßt; und eine Lösung in Kontakt mit dem mit dem transparenten Substrat verbundenen ersten Antikörper; wobei die Lösung einen markierten zweiten Antikörper umfaßt, der bei Anwesenheit eines Toxins in den Säften des Nahrungsmittels an das transparente Substrat gebunden wird, wodurch das Substrat opak wird.

7. Nahrungsmittelverunreinigungs-Detektor nach Anspruch 6, wobei die Lösung, die den zweiten Antikörper umfaßt, in einem abgeteilten Raum eingeschlossen ist.

8. Nahrungsmittelverunreinigungs-Detektor nach Anspruch 7, wobei der abgeteilte Raum eine semipermeable Membran (38) umfaßt.

9. Nahrungsmittelverunreinigungs-Detektor nach einem der Ansprüche 6, 7 oder 8, wobei der erste Antikörper dazu in der Lage ist, eine antigenische Determinante an dem Toxin zu erkennen, welche verschieden ist von der antigenischen Determinante an dem Toxin, die durch den zweiten Antikörper erkannt wird.

10. Nahrungsmittelverunreinigungs-Detektor nach einem der Ansprüche 6 bis 9, umfassend einen nach dem transparenten Substrat (20) ausgerichteten Strichcode (31).

11. Nahrungsmittelverunreinigungs-Detektor nach einem der Ansprüche 6 bis 10, wobei das transparente Substrat ein Loch (18) in einem Nahrungsmittelbehälter (10) dicht verschließt.

12. Nahrungsmittelverunreinigungs-Detektor nach Anspruch 1, wobei der Indikator (34) Markierungen umfaßt, die bei Anwesenheit eines Toxins entfernt werden, wobei der Detektor einen Strichcode (31) umfaßt, der so nach dem Indikator ausgerichtet ist, daß die Kombination der Anwesenheit von Markierungen an dem Indikator und des Strichcodes einen intakten sichtbaren Indikator bildet.

13. Nahrungsmittelverunreinigungs-Detektor nach Anspruch 12, wobei die Markierungen an dem Indikator durch an ein Substrat gebundene markierte Antikörper gebildet sind.

14. Nahrungsmittelverunreinigungs-Detektor nach Anspruch 12 oder 13, wobei eine Entfernung der Markierungen von dem Indikator-Streifen in einem schadhaften Indikator resultiert.

15. Nahrungsmittelverunreinigungs-Detektor nach Anspruch 1, umfassend:
ein an das Substrat (20) gebundenes Toxin; und wobei der markierte Antikörper darauf gedruckt ist, um einen Strichcode zu bilden, so daß, wenn der Detektor mit einem für den Indikator spezifischen Toxin in Kontakt kommt, die markierten Antikörper von dem Substrat entfernt werden, wobei der Strichcode zerstört wird und dadurch die Anwesenheit des Toxins in dem Nahrungsmittel angezeigt wird.

16. Nahrungsmittelverunreinigungs-Detektor nach Anspruch 1 oder 15, wobei das Toxin aus der Gruppe, die aus Chemikalien, pathogenen Organismen oder anderen krankheitsverursachenden toxischen Wirkstoffen besteht, ausgewählt ist.

17. Nahrungsmittelverunreinigungs-Detektor nach Anspruch 15 oder 16, wobei das Substrat (20) eine transparente Membran (20) umfaßt.

18. Nahrungsmittelverunreinigungs-Detektor nach einem der Ansprüche 15, 16 oder 17, wobei der Indikator einen an eine gefärbte Latex-Perle gebundenen Antikörper umfaßt.

19. Nahrungsmittelverunreinigungs-Detektor nach Anspruch 1 oder 18, wobei der Antikörper aus der Gruppe, die aus monoklonalen Antikörpern und polyklonalen Antikörpern besteht, ausgewählt ist.

20. Nahrungsmittelverunreinigungs-Detektor nach einem der Ansprüche 15 bis 19, wobei ein erstes Toxin und ein zweites Toxin an einer ersten Stelle bzw. an einer zweiten Stelle an das Substrat gebunden sind; wobei ein erster Indikator und ein zweiter Indikator an das erste Toxin bzw. das zweite Toxin gebunden sind, wobei der erste Indikator und der zweite Indikator parallele Streifen auf dem Substrat bilden und wobei die Anwesenheit einer Nahrungsmittelprobe, die eine für die Indikatoren spezifische Verunreinigung enthält, bewirkt, daß die Indikatoren frei werden, und das Muster paralleler Streifen auslöscht.

21. Nahrungsmittelverunreinigungs-Detektor nach Anspruch 20, wobei zusätzliche Toxine auf das Substrat gedruckt sind und an die Indikatoren gebunden werden, um einen Strichcode zu bilden, wobei Veränderungen in dem Strichcode mittels eines Strichcode-Abtasters detektierbar sind.

22. Eine Nahrungsmittelerzeugnis-Verpackung, umfassend einen Nahrungsmittelverunreinigungs-Detektor, eine transparente Hülle (14), die den Behälter und das Nahrungsmittelerzeugnis abdeckt; eine erste Erzeugnisinformations-Komponente, die so innerhalb der Hülle angeordnet ist, daß sie von der Außenseite der Verpackung aus sichtbar ist, und ein erstes Substrat umfaßt, auf welches ein oder mehrere erste visuelle Elemente (31) mit einem Toxin-Detektions-Material gemäß Anspruch 1 gedruckt sind; und eine zweite Erzeugnisinformations-Komponente, die außerhalb der Hülle angeordnet ist und ein zweites Substrat umfaßt, welches nach dem ersten Substrat ausgerichtet ist, ohne die ersten visuellen Elemente zu verbergen, und eine Mehrzahl von zweiten visuellen Elementen (35), welche auf das zweite Substrat mit einem Nicht-Toxin-Detektions-Material gedruckt sind.

23. Verpackung nach Anspruch 22, bei der der Behälter (10) ein in einer Bodenplatte desselben ausgebildetes Fenster (18) aufweist, die erste Komponente eine absorbierende Decklage (38) umfaßt, die so dimensioniert ist, daß sie in den Behälter in einer vorbestimmten Stellung relativ zu dem Fenster hineinpaßt, und das erste Substrat mit dem Boden der Decklage in Ausrichtung nach dem Fenster verbunden ist, wenn sich die Decklage in einer vorbestimmten Stellung befindet.

24. Verpackung nach Anspruch 23, bei der das zweite Substrat nach dem Fenster und dem ersten Substrat ausgerichtet ist.

25. Verpackung nach Anspruch 24, bei der das zweite Substrat opak ist und einen oder mehr Ausschnitte aufweist, wobei das eine oder die mehreren visuellen Elemente des ersten Substrats durch den einen oder die mehreren Ausschnitte hindurch sichtbar sind.

26. Verpackung nach einem der Ansprüche 22 bis 25, bei der das erste Element und das zweite Element zusammen einen Strichcode (31) bilden.

27. Verfahren zum Detektieren einer Verunreinigung in einem Nahrungsmittel, umfassend die folgenden Verfahrensschritte:
Anordnen eines Nahrungsmittels, das Nahrungsmittelsäfte erzeugt, in einem Behälter (10); Bilden eines Indikators (16) durch Verbinden eines Antikörpers mit einem Substrat (20); Inkontaktbringen des Indikators (16), dessen Erscheinungsbild sich bei Anwesenheit eines Toxins verändert, mit den Säften in dem Behälter; und Registrieren des Erscheinungsbilds des Indikators, um festzustellen, ob das Nahrungsmittel verunreinigt ist.

28. Verfahren nach Anspruch 27, wobei der Schritt des Inkontaktbringens eines Indikators mit den Säften ein direktes Verheften des Indikators mit dem zu prüfenden Nahrungsmittel umfaßt.

29. Verfahren nach Anspruch 27 oder 28, wobei beim Schritt des Bildens eine gefärbte Latex-Perle als eine Markierung angebracht wird.

30. Verfahren nach einem der Ansprüche 27 bis 29, wobei der Indikator ein Strichcode (31) ist und die Veränderung im Erscheinungsbild des Indikators den Strichcode durch einen Strichcode-Abtaster unlesbar macht.

31. Verfahren nach Anspruch 27, umfassend die folgenden Verfahrensschritte: Binden von Toxinen an das Substrat (20); Drucken markierter Antikörper darauf, um einen Strichcode zu bilden; Inkontaktbringen des Strichcodes mit Material, das von dem Nahrungsmittel stammt, welches im Verdacht steht, mit einem Toxin verunreinigt zu sein; und Abtasten des Strichcodes, um die Anwesenheit von Verunreinigungen in dem Nahrungsmittel festzustellen.

32. Verfahren nach Anspruch 31, wobei die Toxine aus der Gruppe, die aus Chemikalien, pathogenen Organismen oder anderen krankheitsverursachenden toxischen Wirkstoffen besteht, ausgewählt sind.

33. Verfahren nach Anspruch 31 oder 32, wobei das Substrat mit einer transparenten Membran versehen ist.

34. Verfahren nach einem der Ansprüche 31, 32 oder 33, wobei der Indikator einen an eine Latex-Perle gebundenen Antikörper umfaßt.

35. Verfahren nach Anspruch 34, wobei der Antikörper aus der Gruppe, die aus monoklonalen Antikörpern und polyklonalen Antikörpern besteht, ausgewählt ist.

36. Verfahren zum Verpacken eines Nahrungsmittelerzeugnisses, umfassend die folgenden Verfahrensschritte: Bilden eines Indikators (16) durch Drucken eines oder mehrerer sichtbarer Toxin-anzeigender Elemente (16) auf ein erstes Substrat, einschließlich des Unter-Verfahrensschritts des Verbindens eines markierten Antikörpers mit dem Substrat (20), um ein Material zu bilden, welches sich sichtbar verändert, wenn es einem zu detektierenden Toxin ausgesetzt wird; Anordnen des ersten Substrats (20) in dem Nahrungsmittelbehälter; Anordnen eines Nahrungsmittelerzeugnisses in dem Behälter; Einhüllen des Containers und des Erzeugnisses in einen Bogen (14) eines transparenten Materials derart, daß das erste Substrat sichtbar ist; Drucken einer Mehrzahl sichtbarer erzeugnisidentifizierender Elemente auf ein zweites Substrat; und Anordnen des zweiten Substrats an der Außenseite des Bogens in Ausrichtung nach dem ersten Substrat, ohne das eine oder die mehreren Toxin-anzeigenden Elemente zu verbergen.

37. Verfahren nach Anspruch 36, bei dem der Schritt des Anordnens des ersten Substrats in einem Nahrungsmittelbehälter folgendes umfaßt: Anordnen des ersten Substrats an einer Oberfläche einer absorbierenden Deckschicht (38) und Anordnen der einen Oberfläche der Deckschicht in einer vorbestimmten Stellung in Kontakt mit dem Boden des Behälters, und Ausbilden eines Fensters (18) in dem Boden des Behälters in Ausrichtung nach dem ersten Substrat.

## Revendications

1. Détecteur de la contamination d'aliments comprenant un conteneur d'aliment (10) ; un indicateur (16) comprenant un anticorps fixé à un substrat (20) en communication avec les jus provenant de l'aliment ; un moyen (36, 38) de modification de l'aspect de l'indicateur quand une toxine est présente dans les jus de l'aliment.

2. Détecteur de la contamination d'aliments selon la revendication 1, dans lequel le moyen (36, 38) de modification de l'aspect de l'indicateur déclenche une modification de la couleur de l'indicateur.

3. Détecteur de la contamination d'aliments selon la revendication 1 ou 2, dans lequel le moyen (36, 38) de modification de l'aspect de l'indicateur comprend un marqueur fixé à l'anticorps tel que l'anticorps se dissocie du substrat en présence de la toxine.

4. Détecteur de la contamination d'aliments selon la revendication 3, dans lequel le marqueur comprend une bille en latex colorée.

5. Détecteur de la contamination d'aliments selon une quelconque des revendications précédentes, dans lequel l'indicateur est un code à barres (31) ou un symbole devenant illisible quand la toxine est présente.

6. Détecteur de la contamination d'aliments selon la revendication 1, dans lequel l'indicateur comprend un premier anticorps fixé à un substrat transparent (20) et une solution en contact avec le premier anticorps fixé au substrat transparent, la solution comprenant un second anticorps marqué qui se fixe au substrat transparent, rendant ainsi le substrat opaque si une toxine est présente dans les jus de l'aliment.

7. Détecteur de la contamination d'aliments selon la revendication 6, dans lequel la solution comprenant le second anticorps est enfermée dans un compartiment.

8. Détecteur de la contamination d'aliments selon la revendication 7. dans lequel le compartiment comprend une membrane semi-perméable (38).

9. Détecteur de la contamination d'aliments selon la revendication 6, 7 ou 8, dans lequel le premier anticorps est capable de reconnaître sur la toxine un déterminant antigénique diffèrent du déterminant antigénique reconnu sur la toxine par le second anticorps.

10. Détecteur de la contamination d'aliments selon une quelconque des revendications 6 à 9, comprenant un code à barres (31) aligné sur le substrat transparent (20).

11. Détecteur de la contamination d'aliments selon une quelconque des revendications 6 à 10, dans lequel le substrat transparent obture un trou (18) dans le conteneur d'aliment (10).

12. Détecteur de la contamination d'aliments selon la revendication 1, dans lequel l'indicateur (34) comprend des marquages qui disparaissent en présence d'une toxine, le détecteur comprenant un code à barres (31) aligné sur l'indicateur de telle sorte que la combinaison de la présence des marquages sur l'indicateur et du code à barres forme un indicateur visible intact.

13. Détecteur de la contamination d'aliments selon la revendication 12. dans lequel les marquages sur l'indicateur sont formés par des anticorps marqués fixés à un substrat.

14. Détecteur de la contamination d'aliments selon la revendication 12 ou 13, dans lequel la disparition des marquages sur la bandelette de l'indicateur a pour résultat un indicateur défectueux.

15. Détecteur de la contamination d'aliments selon la revendication 1 comprenant une toxine liée au substrat (20) et dans lequel l'anticorps marqué est imprimé sur celui-ci de façon à former un code à barres tel que, quand le détecteur est en contact avec la toxine spécifique de l'indicateur, les anticorps marqués se détachent du substrat, détruisant ainsi le code à barres et indiquant alors la présence de la toxine dans l'aliment.

16. Détecteur de la contamination d'aliments selon la revendication 1 ou 15, dans lequel la toxine est choisie dans le groupe constitué par des produits chimiques, des organismes pathogènes ou d'autres agents toxiques induisant des maladies.

17. Détecteur de la contamination d'aliments selon la revendication 15 ou 16, dans lequel le substrat (20) comprend une membrane transparente (20).

18. Détecteur de la contamination d'aliments selon la revendication 15, 16 ou 17, dans lequel l'indicateur comprend un anticorps fixé sur une bille en latex colorée.

19. Détecteur de la contamination d'aliments selon la revendication 1 ou 18. dans lequel l'anticorps est choisi dans le groupe constitué par les anticorps monoclonaux et les anticorps polyclonaux.

20. Détecteur de la contamination d'aliments selon une quelconque des revendications 15 à 19, dans lequel une première et une seconde toxine sont liées au substrat en une première et une seconde position respectivement ; un premier et un second indicateur sont liés à la première et à la seconde toxine respectivement, le premier et le second indicateur formant des barres parallèles sur le substrat et dans lequel la présence d'un échantillon d'aliment contenant un contaminant spécifique pour les indicateurs entraîne la suppression de la liaison des indicateurs et l'effacement du dessin constitué par les barres parallèles.

21. Détecteur de la contamination d'aliments selon la revendication 20, dans lequel des toxines supplémentaires sont imprimées sur le substrat et fixées aux indicateurs avec formation d'un code à barres dont les modifications sont décelables par un lecteur de codes à barres.

22. Conditionnement d'un produit alimentaire comprenant un détecteur de la contamination d'aliments, un emballage transparent (14) recouvrant le conteneur et le produit alimentaire ; un premier composant d'information sur le produit disposé à l'intérieur de l'emballage de telle façon qu'il soit visible de l'extérieur du conditionnement et comprenant un premier substrat sur lequel un ou plusieurs premiers éléments visuels (31) sont imprimés au moyen d'une matière détectant des toxines selon la revendication 1 et un second composant d'information sur le produit disposé à l'extérieur de l'emballage et comprenant un second substrat aligné sur le premier substrat et ne cachant pas les premiers éléments visuels, et plusieurs seconds éléments visuels (35) imprimés sur le second substrat au moyen d'une matière ne détectant pas les toxines.

23. Conditionnement selon la revendication 22, dans lequel le conteneur (10) comporte une fenêtre (18) ménagée dans son panneau de fond, le premier composant comprend une doublure absorbante (38) dimensionnée de telle façon qu'elle s'ajuste dans le conteneur en une position prédéfinie par rapport à la fenêtre et le premier substrat est fixé au fond de la doublure en alignement sur la fenêtre quand la doublure est dans sa position prédéfinie.

24. Conditionnement selon la revendication 23, dans lequel le second substrat est aligné sur la fenêtre et le premier substrat.

25. Conditionnement selon la revendication 24, dans lequel le second substrat est opaque et a une ou plusieurs ouvertures découpées, un ou plusieurs éléments visuels du premier substrat étant visibles au travers de la ou des découpes.

26. Conditionnement selon une quelconque des revendications 22 à 25. dans lequel le premier et le second élément forment ensemble un code à barres (31).

27. Méthode de détection d'une contamination dans des aliments comprenant les étapes du placement d'un aliment générant des jus alimentaires dans un conteneur (10) ; de la formation d'un indicateur (16) par fixation d'un anticorps à un substrat (20); de la mise en contact, avec les jus dans le conteneur, de l'indicateur (16) dont l'aspect change en présence d'une toxine et de l'observation de l'aspect de l'indicateur pour déterminer si l'aliment est contaminé.

28. Méthode selon la revendication 27, dans laquelle l'étape de la mise en contact d'un indicateur avec les jus comprend la fixation directe de l'indicateur sur l'aliment à tester.

29. Méthode selon la revendication 27 ou 28, dans laquelle dans l'étape de formation, on fixe une bille en latex colorée constituant un marqueur.

30. Méthode selon une quelconque des revendications 27 à 29, dans laquelle l'indicateur est un code à barres (31) et la modification de l'aspect de l'indicateur rend le code à barres illisible par un lecteur de codes à barres.

31. Méthode selon la revendication 27, comprenant les étapes de fixation de toxines sur le substrat (20) ; d'impression d'anticorps marqués sur celles-ci pour former un code à barres ; de misc cn contact du code à barres avec une matière générée par l'aliment suspect d'être contaminé par une toxine et de lecture du code à barres pour déterminer la présence de contaminants dans l'aliment.

32. Méthode selon la revendication 31, dans laquelle les toxines sont choisies dans le groupe constitué par des produits chimiques, des organismes pathogènes ou d'autres agents toxiques induisant des maladies.

33. Méthode selon la revendication 31 ou 32, dans laquelle le substrat est doté d'une membrane transparente.

34. Méthode selon la revendication 31, 32 ou 33, dans laquelle l'indicateur comprend un anticorps fixé à une bille en latex.

35. Méthode selon la revendication 34, dans laquelle l'anticorps est choisi dans le groupe constitué par les anticorps monoclonaux et les anticorps polyclonaux.

36. Méthode de conditionnement d'un produit alimentaire comprenant les étapes ; de la formation d'un indicateur (16) par impression d'un ou plusieurs éléments visibles indicateurs de toxines (16) sur un premier substrat, incluant la sous-étape de la fixation d'un anticorps marqué au substrat (20), pour former un matériau qui se modifie visiblement quand il est soumis à une toxine à détecter; de placement du premier substrat (20) dans un conteneur d'aliment ; de placement d'un produit alimentaire dans le conteneur; d'emballage du conteneur et du produit dans une feuille (14) de matière transparente de telle sorte que le premier substrat soit visible; d'impression de plusieurs éléments visibles identifiant le produit sur un second substrat et d'application du second substrat à l'extérieur de la feuille en alignement sur le premier substrat sans cacher le ou les éléments indicateurs de toxines.

37. Méthode selon la revendication 36, dans laquelle l'étape de placement du premier substrat dans un conteneur d'aliment comprend la fixation du premier substrat sur une surface d'une doublure absorbante (38) et la mise en contact de cette surface de la doublure avec le fond du conteneur dans une position prédéterminée et la formation d'une fenêtre (18) au fond du conteneur en alignement sur le premier substrat.
